# EUROPEAN PATENT APPLICATION

(11) **EP 1 818 055 A1**
(43) Date of publication of application: **15.08.2007**
(21) Application number: 04799946.1
(22) Date of filing: 29.11.2004
(51) Int. Cl.: A61K 31/33

(54) **ATHLETIC ABILITY ENHANCING COMPOSITION**

(71) Applicant: Toyo Shinyaku Co., Ltd., Fukuoka-shi, Fukuoka 812-0011 (JP)
(72) Inventor: TAKAGAKI, Kinya 6th Fl. Kyukan Recruit Hakata Bldg, Fukuoka-shi, Fukuoka 812-0011 (JP); MITSUI, Takeshi 6th Fl. Kyukan Recruit Hakata Bldg, Fukuoka-shi, Fukuoka 812-0011 (JP)
(74) Representative: Smyth, Gyles Darren
(86) International application number: PCT/JP2004/018094
(87) International publication number: WO 2006/057073

(57) **Abstract**

The invention provides a composition including a proanthocyanidin, and preferably further including at least one selected from the group consisting of amino acids, peptides, proteins, and vitamins. The composition of the invention has an excellent effect of protecting the cardiovascular system, such as blood vessels, and also has an excellent effect of enhancing athletic ability, such as enhancement of muscle strength.

## Description

### Technical Field

The present invention relates to a composition for enhancing athletic ability.

### Background Art

Among athletes, an attempt is now actively made to enhance physical athletic ability with dietary and nutritional supply treatment, in addition to the enhancement of muscle strength and improvement of athletic skills through physical training. In particular, an attempt is made to enhance the resistance to fatigue through the intake of elements, such as vitamin and amino acid, that are involved in muscle strength and metabolism (for example, see Japanese Laid-Open Patent Publication Nos. 2002-65212, 2001-69949, and 5-336924).

However, in recent years, there has raised a problem that the physical training in the state of excessive intake of amino acid or vitamin, or unbalanced diet tasks the cardiovascular system, which leads to an athlete has led to sudden death. Also, in our modern society with developed transportation, there has raised a problem that an ordinary person does not get sufficient exercise, causing the decrease in basal metabolism due to decreased athletic ability or lifestyle-related diseases.

In order to avoid the cardiovascular-associated diseases among athletes, as well as to avoid the decreased athletic ability and associated adverse effect among ordinary persons, it is necessary to enhance athletic ability while protecting the cardiovascular system.

### Disclosure of Invention

Accordingly, there is a need for foods and medicines that can enhance athletic ability while protecting the cardiovascular system, and can enhance basal metabolism as well.

The composition for enhancing athletic ability (hereinafter, which is also referred to as "athletic ability enhancing composition") provided according to the invention is characterized in that it includes a proanthocyanidin.

The athletic ability enhancing composition of the invention includes a proanthocyanidin.

In a preferable embodiment, the composition further includes at least one selected from the group consisting of amino acids, peptides, proteins, and vitamins.

By ingesting the athletic ability enhancing composition which includes a proanthocyanidin, an excellent effect can be obtained for protecting the cardiovascular system, such as the blood vessels, as well as enhancing athletic ability, such as enhancement of muscle strength. The composition preferably further includes at least one selected from the group consisting of amino acids, peptides, proteins, and vitamins. The composition of the invention can be applied in the articles such as foods, drugs, and quasi-drugs.

### Brief Description of the Drawings

Fig. 1 is a graph showing the swimming time for the rats that ingested liquid samples of the composition of the invention and a comparative liquid sample, respectively.
Fig. 2 is a graph showing the swimming time for the rats that ingested liquid samples of the composition of the invention and a comparative liquid sample, respectively.
Fig. 3 is a graph showing the modulus of elasticity for the aorta for the rats that ingested feeds of the composition of the invention and basic feed, respectively.
Fig. 4 is a graph showing the soleus muscle weight/body weight for the rats that ingested feeds of the composition of the invention and basic feed, respectively.

### Best Mode for Carrying Out the Invention

The athletic ability enhancing composition of the invention is described below. It should be noted that the following description should not be construed as limiting of the invention, and it will be apparent to those skilled in the art that various modifications may be made to the present invention within the scope of the spirit of the invention.

The athletic ability enhancing composition of the invention contains a proanthocyanidin, and preferably further contains at least one selected from the group consisting of amino acids, peptides, proteins, and vitamins. The composition can contain optionally other components. The components in the composition are as described below.

### Proanthocyanidin

In the present specification, the proanthocyanidin refers to any compounds that are condensation products having flavan-3-ol and/or flavan-3,4-diol as a constituent unit and having a degree of polymerization of 2 or more. Proanthocyanidins are known to have various activities including an antioxidation ability.

Preferably, the proanthocyanidin including a large proportion of condensation products having a low degree of polymerization is used in the present invention. The condensation product having a low degree of polymerization is preferably any condensation products having a degree of polymerization of 2 to 30 (dimer to 30-mer), more preferably having a degree of polymerization of 2 to 10 (dimer to decamer), and even more preferably having a degree of polymerization of 2 to 4 (dimer to tetramer). In this specification, the condensation product having a degree of polymerization of 2 to 4 is referred to as oligomeric proanthocyanidin (hereinafter, OPC). Proanthocyanidins are one kind of polyphenols, and are a potent antioxidant produced by plants, and are contained abundantly in leaves, bark, skin of fruits, or seeds of plants. Specifically, proanthocyanidins, especially OPCs, are contained in bark of plants such as pine, oak, and bayberry; fruits or seeds of grape, blueberry, strawberry, avocado, locust, cowberry, and the like; hull of barley, wheat, soybean, black soybean, cacao, adzuki bean, and conker; inner skin of peanuts; and leaves of ginkgo, for example. It is also known that OPCs are contained in cola nuts in West Africa, the roots of Rathania in Peru, and Japanese green tea. OPCs cannot be produced in the human body.

In particular, a more excellent effect can be obtained for protecting a cardiovascular system and enhancing athletic ability, such as increasing muscular strength, by using the proanthocyanidin having a high OPC content or extracts containing the proanthocyanidin having a high OPC content, compared with the proanthocyanidin having a low OPC content (proanthocyanidin including a large proportion of condensation products having a high degree of polymerization). Further, it is possible that the increase in muscular strength leads to the enhancement of basal metabolism.

Since OPCs are antioxidant, OPCs have an effect of reducing the risk of adult diseases, such as cancers, cardiac diseases, and cerebral thrombosis; an effect of improving allergic diathesis, such as arthritis, atopic dermatitis, and pollenosis, and the like, in addition to the effect of enhancing athletic ability as mentioned above. Furthermore, OPCs also have an effect of inhibiting the proliferation of bacteria in the oral cavity to reduce plaque (dental plaque); an effect of recovering the elasticity of blood vessels; an effect of preventing lipoprotein in blood from being damaged by active oxygen, thereby preventing aggregation and adherence of oxidized lipoprotein onto the inside wall of the vessel, thus preventing cholesterol from being aggregated and adhered onto the oxidized lipoprotein that have been adhered onto the inside wall of the vessel; an effect of regenerating vitamin E that has been degraded by active oxygen; and an effect of serving as an enhancer of vitamin E.

For the proanthocyanidin in the composition according to the present invention, materials such as milled powders of, or extract from the bark, fruits, or seeds of plants as mentioned above can be used. Among them, the extract, in particular from the bark of pine, is preferably used. The extract from the bark of pine has a markedly biological activity among extracts from proanthocyanidin-containing plants. This may be because that the bark of pine contains OPCs abundantly, or contains different effective ingredient(s) in addition to proanthocyanidin. Therefore, the bark of pine is preferably used as the starting material of proanthocyanidin according to the present invention. It is preferable to remove any contaminants from extracts.

Hereinafter, a method for preparing the extract containing mainly proanthocyanidins is described taking, as an example, using the bark of pine, which contains OPCs abundantly, as the starting material.

As the extract from the bark of pine (hereinafter, which is also referred to as "pine bark extract"), any extract from the bark of plants of Pinales, such as French maritime pine (Pinus martima), Larix leptolepis, Pinus thunbergii, Pinus densiflora, Pinus parviflora, Pinus pentaphylla, Pinus koraiensis, Pinus pumila, Pinus luchuensis, utsukushimatsu (Pinus densiflora form. umbraculifera), Pinus palustris, Pinus bungeana, and Anneda in Quebec, Canada, is preferably used. Among them, the extract from the bark of French maritime pine (Pinus martima) is especially preferable.

French maritime pine refers to maritime pines that grow in a part of the Atlantic coastal area in southern France. It is known that the bark of French maritime pine contains proanthocyanidins, organic acids, and other bioactive substances, and has a potent antioxidation ability of removing active oxygen due to proanthocyanidins, which are contained as the main component.

The pine bark extract is obtained by extracting the bark of the pines as mentioned above using water or an organic solvent. When water is used, it is preferable to use warm water or hot water. In order to increase efficiency for the extraction, it is preferable to add a salt such as sodium chloride to water. As the organic solvent used for extraction, an organic solvent acceptable for production of foods or pharmaceuticals can be used. Examples of the organic solvent include methanol, ethanol, 1-propanol, 2-propanol, 1-butanol, 2-butanol, acetone, hexane, cyclohexane, propylene glycol, aqueous ethanol, aqueous propylene glycol, methyl ethyl ketone, glycerin, methyl acetate, ethyl acetate, diethyl ether, dichloromethane, edible oils or fats, 1,1,1,2-tetrafluoroethane, and 1,1,2-trichloroethene. As the solvent for extraction, the water and organic solvents as mentioned above may be used alone or in combination. In particular, water, hot water, ethanol, aqueous ethanol, and aqueous propylene glycol are preferably used. In view of the safety on foods or drugs, water, hot water, ethanol, and aqueous ethanol are more preferably used.

The method for extracting the proanthocyanidin from the park of pine is not particularly limited. For example, a pine bark extract having a highly biological activity and high water solubility can be obtained by extracting with 1 to 50 parts by weight of water at 50 to 120 °C, preferably 70 to 100 °C, with regard to 1 part by weight of dried pine bark. Heat extraction or supercritical fluid extraction can be employed.

The supercritical fluid extraction can be performed with the addition of entrainer. Specifically, the supercritical fluid extraction can be performed using a fluid for extraction prepared by adding an entrainer, such as ethanol, propanol, n-hexane, acetone, toluene, or other aliphatic lower alcohols, aliphatic hydrocarbons, aromatic hydrocarbons, or ketones, at about 2 to 20 W/V% to a supercritical fluid, for dramatically increasing the solubility in a solvent for extraction of a target to be extracted, such as OPCs and catechins (described later), or enhancing the selectivity of separation. Using this method, a pine bark extract can be obtained efficiently.

Since the supercritical fluid extraction can be performed at a relatively low temperature, it has the following advantages: it is applicable for extracting substances that deteriorate or decompose at high temperatures; the fluid for extraction does not remain; and the fluid for extraction can be recovered and recycled so that the steps including a step of removing the extracting fluid can be omitted, and thus, the process can be simplified.

The extraction from the bark of pine can be performed using a batch method using liquid carbon dioxide, a reflux method using liquid carbon dioxide, a reflux method using supercritical carbon dioxide, or the like, other than those mentioned above.

The extraction from the bark of pine can be performed using the combination of a plurality of extraction processes. By combining a plurality of extraction processes, pine bark extracts can be obtained with various compositions.

The pine bark extract prepared as described above can be purified in order to enrich the proanthocyanidin content. Although the purification can be usually performed using an organic solvent such as ethyl acetate, it is preferable to purify the extract using any process without organic solvents, such as ultrafiltration or column or batch system using an adsorption resin (e.g., DIAION HP-20, Sephadex-LH20, and chitin) in view of the safety on foods or drugs.

In the present invention, the pine bark extract that contains mainly proanthocyanidins can be specifically prepared according to a procedure as exemplified below, which is merely an example, and there is no limitation hereto.

First, 1 kg of the bark of French maritime pine is immersed in 3 L of a saturated solution of sodium chloride, and extraction is performed for 30 minutes at 100°C to obtain an extract liquid (extraction step). Then, the extract liquid is filtrated, and the resultant insoluble material is washed with 500 ml of a saturated solution of sodium chloride to obtain a washed liquid (washing step). The extract liquid and the washed liquid are combined to obtain a crude extract liquid of pine bark.

Next, 250 ml of ethyl acetate is added to this crude extract liquid, mixed, and separated to obtain an ethyl acetate layer. This process is repeated additional four times, and the obtained ethyl acetate layers are combined. The resultant ethyl acetate extract is added directly to 200 g of anhydrous sodium sulfate for dehydration, and then filtrated. The filtrated extract is concentrated under a reduced pressure to a volume of 1/5 of the original filtrated extract. The concentrated ethyl acetate extract is poured into 2 L of chloroform and stirred, and the resultant precipitate is recovered by filtration. Subsequently, this precipitate is dissolved in 100 ml of ethyl acetate, and then the resultant solution is added to 1 L of chloroform to form a precipitate. This process is repeated again, and thus, a washing process is accomplished. According to the procedure as mentioned above, for example, about 5 g of pine bark extract containing at least 20 wt% of OPCs and at least 5 wt% of catechins can be obtained.

Preferably, the extract from starting material plants, including the bark of pine, contains at least 40 wt% of proanthocyanidins. It is further preferable that the extract contains at least 15 wt%, more preferably at least 20 wt%, still more preferably at least 30 wt%, of OPCs. For the material containing a large proportion of proanthocyanidins, the pine bark extract is preferably used as mentioned above.

Preferably, the extract from plants, such as the pine bark extract, contains catechins together with proantocyanidins, in particular, OPCs. The term "catechins" is a general term of polyhydroxyflavan-3-ols. As the catechins, (+)-catechin (which is referred to as "catechin" in a narrow sense), (-)-epicatechin, (+)-gallocatechin, (-)-epigallocatechin, epigallocatechin gallate, epicatechin gallate, and afzelechin are known, for example. In addition to (+)-catechin, gallocatechin, afzelechin, 3-galloyl derivatives of (+).catechin, and 3-galloyl derivatives of gallocatechin are isolated from the extract from starting material plants, including the bark of pine. Catechins alone have poor solubility in water and exhibit low bioactivity. In the presence of OPCs, catechins have increased solubility in water and are activated. Therefore, catechins act effectively when ingested together with OPCs.

Catechins are contained at ratio of 5 wt% or more, preferably 10 wt% or more in the above-mentioned plant material extracts. Further preferably, a formulation is prepared so that it contains a plant material extract containing at least 20 wt% of OPCs and at least 5 wt% of catechins. For example, when the extract has a catechin content less than 5 wt%, it is possible to add catechins to the extract so that the catechin content is at least 5 wt% in the extract. It is most preferable to use a pine bark extract containing at least 20 wt% of OPCs and at least 5 wt% of catechins.

### Amino Acids, Peptides, Proteins, and Vitamins

The athletic ability enhancing composition of the invention contains at least one selected from the group consisting of amino acids, peptides, proteins, and vitamins. The component defined above may be prepared by chemical synthesis or extraction from plants or animals. Also, food materials, such as obtained from plants or animals, containing the component defined above can be used. The component defined above, together with a proanthocyanidin, can enhance the effect of proanthocyanidin to protect the cardiovascular system and to enhance athletic ability.

There are no particular limitations regarding the species of amino acids. As amino acids, arginine, which is involved in metabolism in muscle tissue, and glutamic acid, which makes up 50 to 60% in the amino acid composition of skeletal muscle, are preferable. Further, branched amino acids (leucine, isoleucine, and valine) which are involved in basal metabolism are preferable in that they can promote energy metabolism and contribute to athletic ability. As proteins, any proteins (such as wheat germ, soybean protein, collagen, and whey protein) including a high proportion of the amino acids listed above (specifically, arginine, glutamic acid, and branched amino acids) are preferable. As peptides, any hydrolyzates of the protein as mentioned above, for instance, are preferable. The amino acids, peptides, and proteins are nutritional factors essential for enhancement of muscle strength. Therefore, the elements mentioned above, such as amino acids, peptides, or proteins, are important for promoting the event, so-called "super compensation", which occurs on the repair of muscle tissue fatigued due to physical training for the purpose of enhancement of muscle strength. Further, the elements mentioned above, such as amino acids, peptides, or proteins, are necessary for maintaining muscle strength and increasing basal metabolism in ordinary life.

Since vitamins are essential to *in vivo* metabolism, vitamins can be contained, in particular with the aim of enhancing the athletic ability. As vitamins, ascorbic acid and derivatives thereof, and vitamins of vitamin B group, such as vitamin B₁, vitamin B₂ and vitamin B₆, are preferable.

Ascorbic acid and derivatives thereof are important elements for the synthesis of collagen, which is one of constituents of blood vessels or connective tissues. It is known that ascorbic acid is taken in conjunction with OPCs to increase the absorption of ascorbic acid and the duration of physiological activity. Thus, an excellent effect can be obtained for protecting the cardiovascular system, in particular, blood vessels. Ascorbic acid and derivatives thereof are also known to have an effect of reducing stress (in particular, oxidative stress), an anti-thrombus effect, and an effect of enhancing immune strength.

Regarding vitamins of vitamin B group, it is known that vitamin B₁ is a coenzyme involved in carbohydrate metabolism in particular, and deficiency of vitamin B₁ may cause damage to the heart. Vitamin B₁ can synergistically enhance the effect of proanthocyanidin to enhance the athletic ability and protect the cardiovascular system. Vitamin B₂ can promote lipid metabolism in particular to enhance an effect of enhancing athletic ability. Vitamin B₂ has an effect of preventing the production of lipid peroxide, and thus protect against the oxidation of lipids in tissue by active oxygen generated during exercise, so that blood vessels can be prevented from being injured. Vitamin B₆ is involved in amino acid metabolism in particular, and can give an effect of enhancing muscle strength to further enhance an effect of enhancing athletic ability.

### Other Components

The composition of the invention, which contains a proanthocyanidin, can contain optionally various components. Examples of such components include any components (such as excipients, fillers, binders, thickeners, emulsifiers, coloring agents, flavors, nutritional components, or food additives) that can be commonly added in food or pharmaceutical products.

Examples of nutritional components include royal jelly; minerals such as eggshell calcium; sulfur- containing compounds such as taurine, and a compound included in garlic; animal oils and fats such as milk fat, lard, tallow, and fish oil; and plant oils and fats such as soybean oil and rapeseed oil. Examples of food additives include green tea powder, lemon powder, honey, maltitol, lactose, sugar solutions, and seasoning agents.

### Athletic Ability Enhancing Composition

The athletic activity enhancing composition of the invention contains a proanthocyanidin, preferably contains at least one selected from the group consisting of amino acids, peptides, proteins, and vitamins, and optionally contains various additives commonly used in food products.

There are no particular limitations regarding the proanthocyanidin content in the composition of the invention. The proanthocyanidin content may be varied depending on the form of the composition. The composition can contain proanthocyanidins preferably at the ratio of 0.001 wt% to 30 wt%, more preferably 0.001 wt% to 10 wt%. It is also preferable that the composition is formulated to contain 1 to 1000 mg, more preferably from 2 to 500 mg of proanthocyanidins as the adult daily intake.

In a case where the composition of the invention contains at least one selected from the group consisting of amino acids, peptides, proteins, and vitamins, there are no particular limitations regarding the amount contained. Preferably, the composition contains proanthocyanidins and at least one selected from the group consisting of amino acids, peptides, proteins, and vitamins at the weight ratio of the proanthocyanidin component to each element of the other component of 1:0.01 to 1:2000, more preferably 1:0.05 to 1:1000, and even more preferably 1:0.5 to 1:300. The composition containing the proanthocyanidin component and each element of the other component at the above defined weight ratio can synergistically enhance the effect of proanthocyanidin with the effect of each element listed above.

The composition of the invention can be formulated in various forms, such as capsules like hard or soft capsules, tablets, pills, powders (powdered medicine), granules, teabag, candy-like viscous liquid, liquid, and paste, which are commonly used by those skilled in the art, for example as the articles such as foods, drugs, or quasi-drugs, depending on the object. Preferably it is formulated as a sports drink containing a pine bark extract having a high proanthocyanidin content. Depending on the form or personal preference, the composition can be taken as is or dissolved in and drunk with a solvent such as cold water, hot water, or milk, or the components can be leached out of the composition and ingested.

### EXAMPLES

Hereinafter, the present invention will be described by way of examples, but it would be appreciated that the present invention is not limited to the following examples.

### Example 1: Preparation of Liquid Sample

A pine bark extract (trade name: flavangenol, Toyo Shinyaku Co., Ltd.), which has the proanthocyanidin content of 40 wt% (including OPCs at 20 wt% of the extract) and the catechin content of 5 wt%, was dissolved in water at the proportion (wt%) as shown in Table 1 to prepare a liquid sample. Hereinafter, the liquid sample will be referred to as Liquid Sample 1. In Table 1, Component A refers to the proanthocyanidin-containing component, and Component B refers to the component of any of amino acids, peptides, proteins, and vitamins.

### Examples 2 through 7: Preparation of Liquid sample

One or more of the pine bark extract mentioned above, apple polyphenol (proanthocyanidin content of 40 wt%: Nikka Whisky Distilling Co., Ltd.), ascorbic acid, vitamin B₁, vitamin B₂, vitamin B₆, arginine, and glutamic acid (which were from Wako Pure Chemical Industrie, Ltd., respectively), and soybean protein (Fuji Oil Co., Ltd.) were dissolved in water at the respective proportions (wt%) as shown in Table 1 to prepare respective liquid samples. Hereinafter, the liquid samples prepared above will be referred to as Liquid Samples 2 to 7, respectively.

### Comparative Example 1

A liquid sample free from proanthocyanidin (Component A) was prepared by dissolving only Component B listed in Table 1 in water at the proportion (wt%) listed in Table 1. Hereinafter, the liquid sample will be referred to as Liquid Sample 8.

**Table 1**

| | | Liquid Sample 1 | Liquid Sample 2 | Liquid Sample 3 | Liquid Sample 4 | Liquid Sample 5 | Liquid Sample 6 | Liquid Sample 7 | Liquid Sample 8 |
|---|---|---|---|---|---|---|---|---|---|
| | | Example 1 | Example 2 | Example 3 | Example 4 | Example 5 | Example 6 | Example 7 | Comparative Example 1 |
| Component A | Pine Bark Extract | 0.1 | - | 0.1 | 0.1 | 0.1 | 0.1 | 0.1 | - |
| | Apple Polyphenol | - | 0.1 | - | - | - | - | - | - |
| | Vitamin B₁ | - | - | 0.05 | 0.05 | - | - | 0.05 | 0.05 |
| | Vitamin B₂ | - | - | 0.05 | - | - | - | - | - |
| | Vitamin B₆ | - | - | - | 0.05 | - | - | 0.05 | 0.05 |
| Component B | Ascorbic Acid | - | - | - | - | 0.1 | - | 0.1 | 0.1 |
| | Arginine | - | - | - | - | - | 1 | - | 1 |
| | Glutamic Acid | - | - | - | - | - | 1 | - | - |
| | Soybean Protein | - | - | - | - | - | - | 1 | - |

| | | | | | | | | | |
|---|---|---|---|---|---|---|---|---|---|
| *values at wt% | | | | | | | | | |

### Example 8: Athletic Ability Enhancing Effect 1

Three-week-old male SD rats (Kyudo Co., Ltd.) were separated into eight groups of five rats each, and animals of each group were acclimated with basic feed (MF feed: Oriental Yeast Co., Ltd.) for seven days. To one group of the rats, Liquid Sample 1 was forcedly orally administered using a sonde at a rate of 1 mL per day for 28 days. During the administration period, the rats were allowed to voluntarily take the basic feed and water.

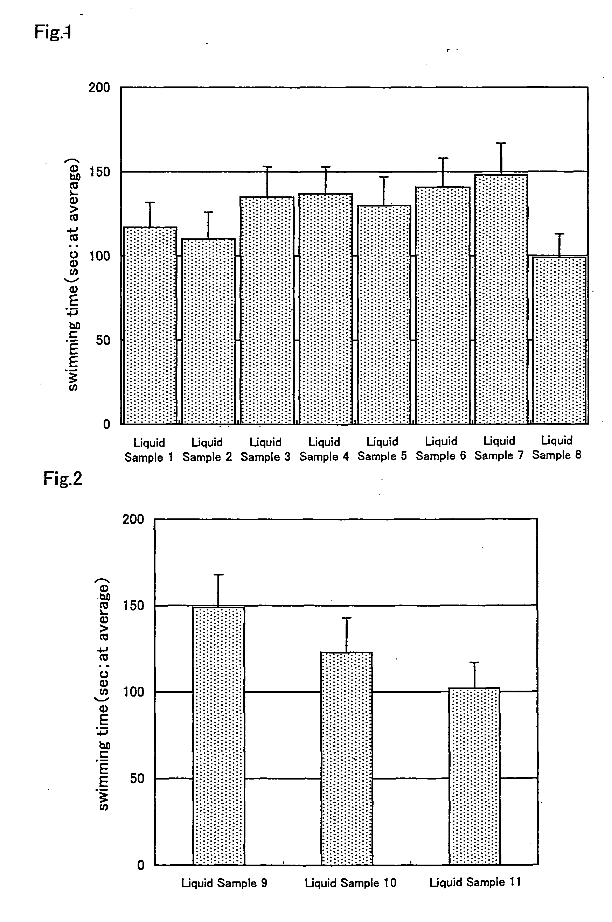

One hour after the administration on the 28th day, the rats were evaluated for athletic ability. The evaluation was carried out by the swimming performance test based on the method of Tanaka et al. (Taiikugaku kenkyu (Jap. J. Phys. Educ.), 33(2), 155, 1988). Specifically, since the 21st day of administration, the rats were subjected to swimming for ten minutes per day in order to be familiarized with swimming. Then, on the 28th day of the experiment, after the tail of each rat was loaded with a weight equivalent to 4 weight percent of the body weight of the rat, the rat was subjected to swimming, and then evaluated for athletic ability by measuring the time for swimming (swimming time), which is the time from the start of swimming to all-out, the point of submerged below the water surface for 10 seconds. Likewise, to the remaining seven groups of the rats, Liquid Samples 2 through 8 were orally administered, respectively, and the rats were evaluated for athletic ability. The results are shown in Fig. 1.

From the results of Fig. 1, it can be found that animals of the group administered the proanthocyanidin-containing liquid sample of the example (such as Liquid Samples 1 through 7) have a longer swimming time than the group which ingested the proanthocyanidin-free liquid sample of the comparative example (such as Liquid Sample 8). From this, it can be understood that the composition that contains a proanthocyanidin has an effect of enhancing the stamina. Accordingly, the composition of the invention has an excellent effect for enhancing athletic ability. In particular, it can be found that an even higher effect is obtained for enhancing athletic ability, in the liquid sample which contains a proanthocyanidin and at least one selected from the group consisting of amino acids, peptides, proteins, and vitamins (such as Liquid Samples 3 through 7). Moreover, it can be found from comparing Example 1 (Liquid Sample 1) with Example 2 (Liquid Sample 2) that a greater effect is obtained for enhancing athletic ability, by using the pine bark extract than apple polyphenol as the source of proanthocyanidin.

### Example 9: Athletic Ability Enhancing Effect 2

An aqueous solution containing 0.1 wt% of the pine bark extract used in Example 1, 5 wt% of valine (Wako Pure Chemical Industries), 5 wt% of leucine (Wako Pure Chemical Industries), and 5 wt% of isoleucine (Wako Pure Chemical Industries); an aqueous solution containing 0.1 wt% of the pine bark extract; and water were prepared as Liquid Sample 9, Liquid Sample 10 and Liquid Sample 11, respectively. In the same manner as in Example 8, the rat was evaluated for athletic ability after administration of the liquid sample. The results are shown in Fig. 2.

From the results of Fig. 2, it can be found that animals of the respective groups administered any of the proanthocyanidin-containing liquid samples (Liquid Samples 9 and 10) have a longer swimming time than the group which ingested water (Liquid Sample 11) as a comparative example. In particular, an even higher effect was observed in the group administered Liquid Sample 9, which contains proanthocyanidins (pine bark extract) and the branched amino acids, valine, leucine and isoleucine, for enhancing athletic ability.

### Example 10: Preparation of Feed

The pine bark extract was added and blended into 0.5 wt% in basic feed (powdered MF: Oriental Yeast Co., Ltd.) to prepare Feed 1. The composition of Feed 1 is shown in Table 2.

### Example 11: Preparation of Feed

Instead of the pine bark extract of Example 10, apple polyphenol was used to prepare Feed 2 in the same manner as in Example 10. The composition of Feed 2 is shown in Table 2.

### Example 12: Preparation of Feed

In addition to the pine bark extract of Example 10, ascorbic acid also was added and blended into 0.5 wt% in the basic feed to prepare Feed 3 in the same manner as in Example 10. The composition of Feed 3 is shown in Table 2.

**Table 2**

| | | Feed1 | Feed2 | Feed3 |
|---|---|---|---|---|
| | | Example10 | Example11 | Example12 |
| Component A | Pine Bark Extract | 0.5 | - | 0.5 |
| | Apple Polyphenol | - | 0.5 | - |
| Component B | Ascorbic Acid | - | - | 0.5 |

| | | | | |
|---|---|---|---|---|
| *values at wt% | | | | |

### Example 13: Evaluation for Blood Vessel Protecting Effect and Muscle Strength Enhancement Effect

Four-week-old male SHR rats (Charles River Laboratories Japan, Inc.) were acclimated with basic feed (powdered MF: Oriental Yeast Co., Ltd.) and water for a week. These rats were separated into groups of five rats each in such a manner that the average weights of rats were substantially equal among the groups. The rats of each group were allowed to voluntarily take any of Feeds 1 to 3 for 28 days. The group of rats that were allowed to voluntarily take the basic feed was used as a control group. It should be noted that all groups were allowed to voluntarily take an aqueous solution containing 1 wt% of NaCl from the day of the start of ingestion.

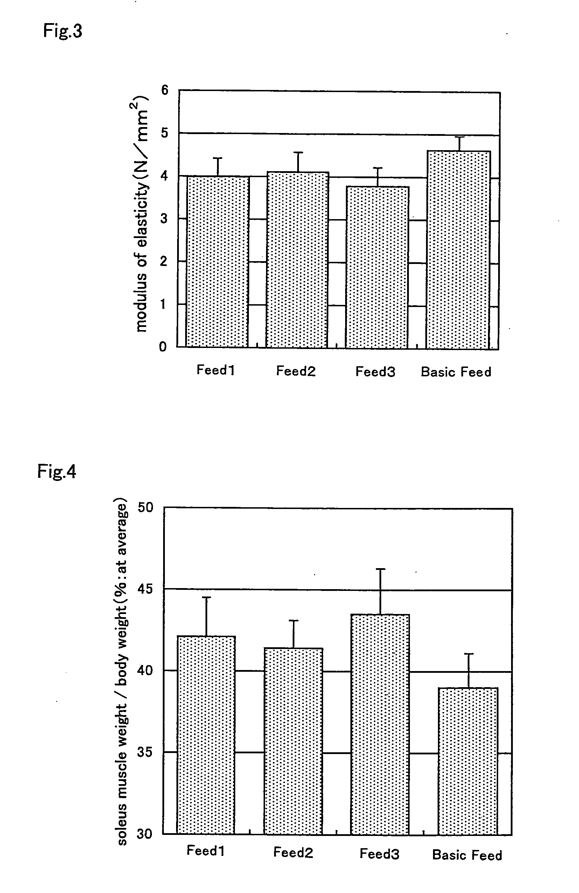

The weight was measured for the respective rats in each group on the 28th day from the start of ingestion. Next, the thoracic aorta was removed and the modulus of elasticity of the removed aorta was determined. The thoracic aorta was pulled using a tension tester (EZ-test, Shimadzu Corporation) for measurement at a crosshead speed of 2 mm/min until rupture, to prepare a stress-deformity curve, and then, a slope was determined from the curve according to the method of least squares, and the slope was regarded as the modulus of elasticity. The results are shown in Fig. 3. It should be noted that the lower the value of the modulus of elasticity, the higher the elasticity of the blood vessel.

Next, the rats of each group were evaluated for an effect of enhancing the muscle strength by removing the soleus muscle from the hind legs of the rat, measuring the weight of the removed soleus muscle, and calculating the ratio of the weight of the-removed soleus muscle to the body weight of the rat (soleus muscle weight / body weight). The results are shown in Fig. 4.

As shown from the results of Fig. 3, lower modulus of elasticity and higher blood vessel flexibility was observed in the group that ingested the proanthocyanidin-containing feed, such as Feeds 1 through 3, compared with in the control group. This indicates that an effect of protecting blood vessels was obtained. In particular, it can be found that an even greater effect was obtained in the group that ingested Feed 3, which contains proanthocyanidins and ascorbic acid.

As shown from the results of Fig. 4, increased muscle weight was observed in the group that ingested the proanthocyanidin-containing feed, such as Feeds 1 through 3. This indicates that the feed has an effect of enhancing the muscle strength. As from the results of Fig. 3, in particular, it can be found that an even greater effect was obtained in the group that ingested Feed 3, which contains proanthocyanidins and ascorbic acid.

The results of Figs. 1 through 4 demonstrate that the proanthocyanidin-containing composition has an effect of enhancing athletic ability, such as stamina enhancement action and muscle strength enhancement action, and also has an effect of protecting the cardiovascular system. In Example 13, thermography was used to measure the surface temperature of the rats before dissection, and an elevated surface temperature was observed compared with before administration of the feed, suggesting that the increase in muscle strength through the ingestion of proanthocyanidin can be caused by an increase in basal metabolism.

### Example 14: Production of Tablets

The following components were mixed to prepare 200-mg tablets.

| Tablet Components | Weight (per 200 mg) |
|---|---|
| pine bark extract | 20 mg |
| collagen | 50 mg |
| soybean protein | 50 mg |
| vitamin mix^{*1} | 25 mg |
| crystalline cellulose | 10 mg |
| sucrose ester | 5 mg |
| silicon dioxide | 2 mg |
| dolomite^{*2} | 38 mg |

| | |
|---|---|
| ^{*}1: containing vitamins of vitamin B group (The Nippon Koryo Yakuhin Kaisha) ^{*}2: containing calcium and magnesium compounds (Sankyo Foods) | |

### Example 15: Production of Sports Drinks

The following components were blended to prepare sports drinks.

| Sports Drink Components | Weight (per 1 L) |
|---|---|
| fructose/glucose liquid sugar | 20 g |
| lemon juice | 10 g |
| citric acid | 2 g |
| L-ascorbic acid | 2 g |
| pine bark extract | 25 mg |
| flavor | 150 mg |
| vitamin B₁ | 0.8 mg |
| vitamin B₂ | 1.2 mg |
| vitamin B₆ | 1.8 mg |
| glutamic acid | 200 mg |
| arginine | 20 mg |
| valine | 200 mg |
| leucine | 100 mg |
| isoleucine | 100 mg |
| pure water | remainder |

### Industrial Applicability

The athletic ability enhancing composition of the invention contains a proanthocyanidin, and exerts an excellent effect of protecting the cardiovascular system such as blood vessels and also exerts an excellent effect of enhancing athletic ability, such as enhancement of muscle strength. In addition, since most of energy metabolism in the body takes place by the muscles, it is possible that an effect of enhancing basal metabolism is obtained as well. The composition of the invention can be applied in the articles such as foods, drugs, and quasi-drugs.

## Claims

1. A composition for enhancing athletic ability, comprising a proanthocyanidin.

2. The composition according to claim 1, further comprising:
at least one selected from the group consisting of amino acids peptides proteins and vitamins.
